# NOUVEAU FASCICULE DE BREVET EUROPEEN

(11) **EP 0 865 271 B2**
(45) Date de publication et mention de la décision concernant l'opposition: **17.03.2004**
(45) Mention de la délivrance du brevet: 19.01.2000
(21) Numéro de dépôt: 96941708.8
(22) Date de dépôt: 05.12.1996
(51) Int. Cl.: A61K 7/42

(54) **Utilisation de l' alpha-cyano-béta, béta-diphénylacrylate de 2-éthylhexyle en vue d' améliorer la stabilité à la lumière du p-méthyl benzylidène camphre**
Verwendung von 2-Ethylhexyl-alpha-cyano-beta, beta-diphenylacrylat zur Verbesserung der Lichtstabilität von p-Methylbenzylidencampher
Use of 2-ethylhexyl alpha-cyano-beta,beta-diphenylacrylate in order to improve the photostability of p-methylbenzylidene camphor

(30) Priorité: 08.12.1995 FR 9514579
(43) Date de publication de la demande: 23.09.1998
(73) Titulaire: L'OREAL, 75008 Paris (FR)
(72) Inventeur: HANSENNE, Isabelle, F-75017 Paris (FR); JOSSO, Martin, F-75006 Paris (FR)
(74) Mandataire: Bulle, Françoise
(86) Numéro de dépôt international: PCT/FR1996/001945
(87) Numéro de publication internationale: WO 1997/021422

(56) Documents cités:
- EP-A- 0 678 292
- EP-A- 0 685 225
- EP-A- 0 685 228
- WO-A-91/11989
- WO-A-94/04131
- DE-A- 3 741 420

## Description

La présente invention concerne l'utilisation d'α-cyano-β,β-diphénylacrylate de 2-éthylhexyle, dans ou pour la fabrication de compositions cosmétiques contenant le p-méthyl-benzylidène camphre en association avec au moins un dérivé de dibenzoylméthane dans le but d'améliorer la stabilité à la lumière du p-méthyl-benzylidène camphre au sein de telles compositions.

On sait que les radiations lumineuses de longueurs d'onde comprises entre 280 nm et 400 nm permettent le brunissement de l'épiderme humain et que les rayons de longueurs d'onde comprises entre 280 nm et 320 nm, connus sous la dénomination d'UV-B, provoquent des érythèmes et des brûlures cutanées qui peuvent nuire au développement du bronzage naturel ; ce rayonnement UV-B doit donc être filtré.

On sait également que les rayons UV-A, de longueurs d'onde comprises entre 320 nm et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions phototoxiques ou photo-allergiques. Il est donc souhaitable de filtrer aussi le rayonnement UV-A.

A cet égard, les dérivés du dibenzoylméthane, et tout particulièrement le 4-tert-butyl-4'-méthoxydibenzoyl-méthane, vendu sous la dénomination commerciale « Parsol 1789 » par la société Givaudan, sont des filtres UV-A particulièrement intéressants compte tenu de leur fort pouvoir d'absorption intrinsèque.

Cependant, ces filtres présentent l'inconvénient d'être photoréactifs, c'est-à-dire qu'ils se dégradent sous l'action de la lumière et perdent par là-même leurs propriétés photoprotectrices de la peau et des cheveux.

La Demanderesse a déjà proposé dans la demande de brevet FR-2 607 700 d'associer au 4-tert-butyl-4'-méthoxydibenzoylméthane du p-méthylbenzylidène camphre afin d'améliorer la stabilité à la lumière des compositions cosmétiques contenant du 4-tert-butyl-4'-méthoxydibenzoyl-méthane.

Toutefois, cette solution n'est pas totalement satisfaisante, compte tenu du fait que le p-méthylbenzylidène camphre est lui-même sujet à une disparition progressive sous l'action de la lumière. Les compositions cosmétiques le contenant voient la concentration en p-méthylbenzylidène camphre baisser sous l'action de la lumière et perdent donc de leur efficacité photoprotectrice.

Or, à la suite d'importantes recherches menées dans le domaine de la photoprotection évoqué ci-dessus, la Demanderesse a maintenant découvert que l'introduction d'α-cyano-β,β-diphénylacrylate de 2-éthylhexyle dans une composition contenant du p-méthyl-benzylidène camphre en association avec un au moins un dérivé de dibenzoylméthane, et en particulier avec le 4-tert-butyl4'-méthoxydibenzoylméthane, permettait d'améliorer de façon significative la stabilité du p-méthyl-benzylidène camphre au sein de telles compositions, et donc l'efficacité globale de ces compositions.

Cette découverte est à la base de l'invention.

La présente invention a donc pour objet l'utilisation d'α-cyano-β,β-diphénylacrylate de 2-éthylhexyle dans, ou pour la fabrication de, compositions cosmétiques comprenant du p-méthyl-benzylidène camphre et un dérivé de dibenzoylméthane, en particulier le 4-tert-butyl-4'-méthoxydibenzoylméthane, en vue d'améliorer dans lesdites compositions la stabilité à la lumière du p-méthyl-benzylidène camphre.

Ainsi, selon la présente invention, on peut réaliser des compositions cosmétiques contenant au moins un dérivé de dibenzoylméthane en association avec le p-méthyl-benzylidène camphre, dont la concentration en p-méthyl-benzylidène camphre reste relativement constante même si ces compositions sont soumises à l'action de la lumière.

D'autres caractéristiques, aspects et avantages de la présente invention apparaîtront à la lecture de la description détaillée qui va suivre.

Comme indiqué précédemment, les dérivés du dibenzoylméthane visés par la présente invention sont des produits déjà bien connus en soi et décrits notamment dans les documents FR-A- 2 326 405, FR-A- 2 440 933 et EP-A- 0 114 607, documents dont les enseignements sont, pour ce qui touche à la définition même de ces produits, totalement inclus à titre de références dans la présente description.

Parmi les dérivés du dibenzoylméthane plus particulièrement visés par la présente invention, on peut notamment citer, de manière non limitative :
- le 2-méthyldibenzoylméthane,
- le 4-méthyldibenzoylméthane,
- le 4-isopropyldibenzoylméthane,
- le 4-tert.-butyldibenzoylméthane,
- le 2,4-diméthyldibenzoylméthane,
- le 2,5-diméthyldibenzoylméthane,
- le 4,4'-diisopropyldibenzoylméthane,
- 4-tert-butyl-4'-méthoxydibenzoylméthane,
- le 2-méthyl-5-isopropyl-4'-méthoxydibenzoylméthane,
- le 2-méthyl-5-tert.-butyl-4'-méthoxydibenzoylméthane,
- le 2,4-diméthyl-4'-méthoxydibenzoylméthane,
- le 2,6-diméthyl-4-tert.-butyl-4'-méthoxydibenzoylméthane.

Parmi les dérivés du dibenzoylméthane mentionnés ci-dessus, on préfère tout particulièrement, selon la présente invention, mettre en oeuvre le 4-tert-butyl-4'-méthoxydibenzoylméthane, notamment celui proposé à la vente sous la dénomination commerciale de "PARSOL 1789" par la Société Givaudan, ce filtre répondant à la formule développée (I) suivante :

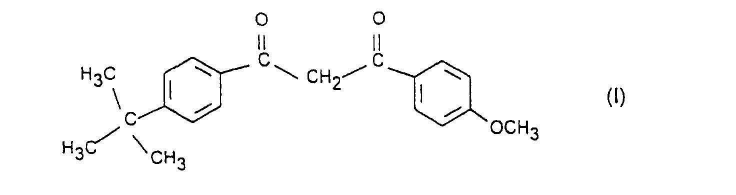

Un autre dérivé du dibenzoylméthane préféré selon la présente invention est le 4-isopropyldibenzoylméthane, filtre vendu sous la dénomination de "EUSOLEX 8020" par la Société Merck, et répondant à la formule développée (II) suivante:

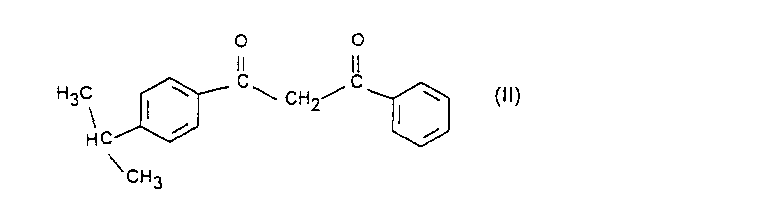

Le p-méthylbenzylidène camphre est un filtre liposoluble connu en soi, absorbant dans l'UV-B et vendu notamment sous la dénomination commerciale « EUSOLEX 6300 » par la société Merck.

L'α-cyano-β,β-diphénylacrylate de 2-éthylhexyle, encore appelé octocrylène, est quant à lui un filtre lipophile liquide déja connu en soi pour son activité dans l'UV-B. Il s'agit d'un produit qui est disponible commercialement, et est vendu notamment sous la dénomination de "UVINUL N 539" par la Société BASF. Il répond à la formule (III) suivante : 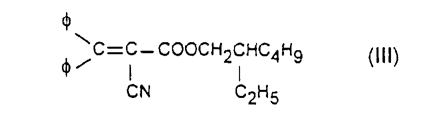 dans laquelle φ désigne un radical phényle.

Ainsi, lorsqu'on ajoute en quantité suffisante de l'α-cyano-β,β-diphénylacrylate de 2-éthylhexyle à une composition contenant du p-méthyl-benzylidène camphre et un dérivé de dibenzoylméthane, en particulier le 4-tert-butyl-4'-méthoxydibenzoylméthane, on observe une augmentation de la stabilité de cette composition à la lumière, et donc une amélioration de son efficacité au cours du temps.

De préférence, l'α-cyano-β,β-diphénylacrylate de 2-éthylhexyle est utilisé selon l'invention à une teneur au moins égale à 0,5 % en poids, par rapport au poids total de la composition. De préférence encore, cette teneur est comprise entre 0,5 % et 3 % en poids, par rapport au poids total de la composition.

Les compositions cosmétiques obtenues selon la présente invention peuvent bien entendu contenir un ou plusieurs filtres solaires complémentaires actifs dans l'UVA et/ou l'UVB (absorbeurs), hydrophiles ou lipophiles, autres bien sûr que les trois filtres mentionnés ci-avant. Ces filtres complémentaires peuvent être notamment choisis parmi les dérivés cinnamiques, les dérivés salicyliques, les dérivés du camphre autres que le p-méthyl-benzylidène camphre comme par exemple l'acide benzène 1,4-di(3-méthylidène-10-camphosulfonique), les dérivés de triazine, les dérivés de la benzophénone, les dérivés de β,β-diphénylacrylate autres que l'α-cyano-β,β-diphénylacrylate de 2-éthylhexyle, les dérivés de l'acide p-aminobenzoïque, les polymères filtres et silicones filtres décrits dans la demande WO-93/04665. D'autres exemples de filtres organiques sont donnés dans la demande de brevet EP-A 0 487 404.

Les compositions cosmétiques peuvent également contenir des agents de bronzage et/ou de brunissage artificiels de la peau (agents autobronzants), tels que par exemple de la dihydroxyacétone (DHA).

Les compositions cosmétiques peuvent encore contenir des pigments ou bien encore des nanopigments (taille moyenne des particules primaires : généralement entre 5 nm et 100 nm, de préférence entre 10 et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs bien connus en soi agissant par blocage physique (réflection et/ou diffusion) du rayonnement UV. Des agents d'enrobage classiques sont par ailleurs l'alumine et/ou le stéarate d'aluminium. De tels nanopigments d'oxydes métalliques, enrobés ou non enrobés, sont en particulier décrits dans les demande de brevets EP-A- 0 518 772 et EP-A- 0 518 773.

Les compositions obtenues selon la présente invention peuvent comprendre en outre des adjuvants cosmétiques classiques notamment choisis parmi les corps gras, les solvants organiques, les épaississants ioniques ou non ioniques, les adoucissants, les antioxydants, les opacifiants, les stabilisants, les émollients, les silicones , les α-hydroxyacides, les agents anti-mousse, les agents hydratants, les vitamines, les parfums, les conservateurs, les tensioactifs, les charges, les séquestrants, les polymères, les propulseurs, les agents alcalinisants ou acidifiants, les colorants, ou tout autre ingrédient habituellement utilisé en cosmétique, en particulier pour la fabrication de compositions antisolaires sous forme d'émulsions.

Bien entendu, l'homme de l'art veillera à choisir le ou les éventuels composés complémentaires cités ci-dessus et/ou leurs quantités de manière telle que les propriétés avantageuses attachées intrinsèquement à l'association ternaire ne soient pas, ou substantiellement pas, altérées par la ou les adjonctions envisagées.

Les corps gras peuvent être constitués par une huile ou une cire ou leurs mélanges, et ils comprennent également les acides gras, les alcools gras et les esters d'acides gras. Les huiles peuvent être choisies parmi les huiles animales, végétales, minérales ou de synthèse et notamment parmi l'huile de vaseline, l'huile de paraffine, les huiles de silicone, volatiles ou non, les isoparaffines, les poly-α-oléfines, les huiles fluorées et perfluorées. De même, les cires peuvent être choisies parmi les cires animales, fossiles, végétales, minérales ou de synthèse connues en soi.

Parmi les solvants organiques, on peut citer les alcools et polyols inférieurs.

Les épaississants peuvent être choisis notamment parmi les acides polyacryliques réticulés, les gommes de guar et celluloses modifiées ou non telles que la gomme de guar hydroxypropylée, la méthylhydroxyéthylcellulose et l'hydroxypropylméthyl cellulose.

Les compositions obtenues selon l'invention peuvent être préparées selon les techniques bien connues de l'homme de l'art, en particulier celles destinées à la préparation d'émulsions de type huile-dans-eau ou eau-dans-huile.

Ces compositions peuvent se présenter en particulier sous forme d'émulsion, simple ou complexe (H/E, E/H, H/ E/H ou E/H/E) telle qu'une crème, un lait, un gel ou un gel crème, de poudre, de bâtonnet solide et éventuellement être conditionnée en aérosol et se présenter sous forme de mousse ou de spray.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR 2 315 991 et FR 2 416 008).

La composition cosmétique obtenue selon l'invention peut être utilisée comme composition protectrice de l'épiderme humain ou des cheveux contre les rayons ultraviolets, comme composition antisolaire ou comme produit de maquillage.

Lorsque la composition cosmétique obtenue selon l'invention est utilisée pour la protection de l'épiderme humain contre les rayons UV, ou comme composition antisolaire, elle peut se présenter sous forme de suspension ou de dispersion dans des solvants ou des corps gras, sous forme de dispersion vésiculaire non ionique ou encore sous forme d'émulsion, de préférence de type huile-dans-eau, telle qu'une crème ou un lait, sous forme de pommade, de gel, de gel crème, de bâtonnet solide, de stick, de mousse aérosol ou de spray.

Lorsque la composition cosmétique obtenue selon l'invention est utilisée pour la protection des cheveux, elle peut se présenter sous forme de shampooing, de lotion, de gel, d'émulsion, de dispersion vésiculaire non ionique, de laque pour cheveux et constituer par exemple une composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.

Lorsque la composition est utilisée comme produit de maquillage des cils, des sourcils ou de la peau, tel que crème de traitement de l'épiderme, fond de teint, bâton de rouge à lèvres, fard à paupières, fard à joues, mascara ou ligneur encore appelé "eye liner", elle peut se présenter sous forme solide ou pâteuse, anhydre ou aqueuse, comme des émulsions huile dans eau ou eau dans huile, des dispersions vésiculaires non ioniques ou encore des suspensions.

A titre indicatif, pour les formulations antisolaires qui présentent un support de type émulsion huile-dans-eau, la phase aqueuse (comprenant notamment les filtres hydrophiles) représente généralement de 50 à 95 % en poids, de préférence de 70 à 90 % en poids, par rapport à l'ensemble de la formulation, la phase huileuse (comprenant notamment les filtres lipophiles) de 5 à 50 % en poids, de préférence de 10 à 30 % en poids, par rapport à l'ensemble de la formulation, et le ou les (co)émulsionnant(s) de 0,5 à 20 % en poids, de préférence de 2 à 10% en poids, par rapport à l'ensemble de la formulation.

Un exemple concret, mais nullement limitatif, illustrant l'invention, va maintenant être donné.

### EXEMPLE:

On a réalisé six émulsions huile-dans-eau de composition suivante :
- p-méthyl-benzylidène camphre (Eusolex 6300) 4,5 %
- 4-tert-butyl-4'-méthoxydibenzoylméthane (Parsol 1789) 1,5 %
- α-cyano-β,β-diphénylacrylate de 2-éthylhexyle (Uvinul N 539) X %
- mélange mono-stéarate de glycérol / stéarate de PEG (100 OE) vendu sous la dénomination commerciale « Arlacel 165 » par la société ICI 1,5 %
- alcool cétylique pur (C16 90 %) bidistillé 1,7 %
- acide polyacrylique réticulé 0,2 %
- benzoate d'alcools C12/C15 8 %
- hexadécylphosphate de potassium 1 %
- triéthanolamine 0,2 %
- acide éthylène diamine tétracétique, sel disodique, 2 H₂O 0,1 %
- cyclométhicone vendue sous la dénomination « DC 245 Fluid » par la société Dow Corning 5 %
- mélange poly diméthylsiloxane alpha-omega dihydroxyle/cyclotetra et cyclopenta diméthylsiloxane (56/44) (14/86) 5%
- antioxydant 0,5 %
- hydratants 15%
- conservateurs qs
- eau déminéralisée stérilisée qsp 100,0%
en faisant varier X de 0 à 10 %.

Pour chacune d'entre elles, on a déterminé le pourcentage de p-méthyl-benzylidène camphre résiduel après irradiation par des UV selon le protocole suivant : pour chaque formule, on a préparé quatre échantillons témoins et quatre échantillons tests. On a déposé sur des plaques de PMMA (polyméthyl méthacrylate) dépolies, préalablement rincées à l'eau puis séchées, 16 mg de formule qu'on a étalée sur une surface de 2 x 4 cm². On a ensuite laissé reposer toutes les plaques une demi-heure à l'obscurité. Puis on a irradié les plaques (SUNTEST CPS Heraeus) pendant 4 heures et 5 minutes en conservant les plaques témoins à l'obscurité pendant le temps d'irradiation des autres plaques.

On a ensuite dosé les échantillons de la manière suivante : on a procédé à l'extraction des filtres en immergeant chaque plaque dans 50 g d'isopropanol afin de solubiliser les filtres. Les plaques et le solvant contenant les filtres ont ensuite été traités aux ultrasons pendant 5 minutes pour assurer une agitation efficace. La concentration totale en filtre résiduel UV-B (Uvinul N 539 + Eusolex 6300) a été dosée au spectrophotomètre.

L'α-cyano-β,β-diphénylacrylate de 2-éthylhexyle étant quant à lui parfaitement photostable (aucune dégradation significative), on a retranché de cette concentration totale en UV-B la concentration initiale en α-cyano-β,β-diphénylacrylate de 2-éthylhexyle : on a ainsi obtenu la concentration en p-méthyl-benzylidène camphre (Eusolex 6300) résiduel.

La concentration en filtre UV-A résiduel (Parsol 1789) a également été mesurée.

Les résultats en pourcentage de filtre résiduel sont consignés dans le tableau (I) suivant :

**Tableau (I)**

| **% d'Uvinul N 539 ajouté** | **% d'Eusolex 6300 résiduel (UV B)** | **% de Parsol 1789 résiduel (UV A)** |
|---|---|---|
| 0 % | 65% | 62 % |
| 0,5% | 84 % | 78 % |
| 1,5% | 76% | 74% |
| 3% | 68 % | 87 % |
| 6% | 79 % | 87 % |
| 10% | 88 % | 92% |

Ces résultats montrent clairement que la présence d'α-cyano-β,β-diphénylacrylate de 2-éthylhexyle (Uvinul N 539) dans une composition contenant du p-méthyl-benzylidène camphre (Eusolex 6300) en association avec du 4-tert-butyl-4'-méthoxydibenzoylméthane (Parsol 1789) augmente la stabilité du p-méthylbenzylidène camphre. Dès l'ajout de 0,5 % d'α-cyano-β,β-diphénylacrylate de 2-éthylhexyle dans une composition contenant 4,5 % de p-méthyl-benzylidène camphre et 1,5 % de 4-tert-butyl-4'-méthoxydibenzoylméthane, la stabilité du p-méthyl-benzylidène camphre est améliorée de façon significative. Compte tenu du fait que le p-méthyl-benzylidène camphre photostabilise le 4-tert-butyl-4'-méthoxydibenzoylméthane (cf. FR-2 607 700), on constate par voie de conséquence (colonne 3 du tableau (I)) que la photostabilité du 4-tert-butyl-4'-méthoxydibenzoylméthane est elle-même nettement améliorée.

La composition obtenue selon l'invention est donc globalement particulièrement photostable.

## Revendications

1. Utilisation de l'α-cyano-β,β-diphénylacrylate de 2-éthylhexyle dans, ou pour la fabrication de, compositions cosmétiques comprenant du p-méthyl-benzylidène camphre et un dérivé de dibenzoylméthane, en vue d'améliorer dans lesdites compositions la stabilité à la lumière du p-méthyl-benzylidène camphre.

2. Utilisation selon la revendication 1, **caractérisée par le fait que** ledit dérivé de dibenzoylméthane est le 4-tert-butyl-4'-méthoxydibenzoylméthane.

3. Utilisation selon la revendication 1, **caractérisée par le fait que** ledit dérivé de dibenzoylméthane est le 4-isopropyldibenzoylméthane.

4. Utilisation selon l'une quelconque des revendications 1 à 3, **caractérisée par le fait que** ledit α-cyano-β,β-diphénylacrylate de 2-éthylhexyle est présent dans ladite composition à une teneur au moins égale à 0,5 % en poids, par rapport au poids total de la composition.

5. Utilisation selon la revendication 4, **caractérisée par le fait que** ladite teneur est comprise entre 0,5 et 3% en poids, par rapport au poids total de la composition.

## Claims

1. Use of 2-ethylhexyl α-cyano-β,β-diphenylacrylate in, or for the manufacture of, cosmetic compositions comprising p-methylbenzylidenecamphor and a dibenzoylmethane derivative, in order to improve the light stability of p-methylbenzylidenecamphor in the said compositions.

2. Use according to Claim 1, **characterized in that** the said dibenzoylmethane derivative is 4-tert-butyl-4'-methoxydibenzoylmethane.

3. Use according to Claim 1, **characterized in that** the said dibenzoylmethane derivative is 4-isopropyldibenzoylmethane.

4. Use according to any one of Claims 1 to 3, **characterized in that** the said 2-ethylhexyl α-cyano-β,β-diphenylacrylate is present in the said composition at a content at least equal to 0.5% by weight relative to the total weight of the composition.

5. Use according to Claim 4, **characterized in that** the said content is between 0.5% and 3% by weight relative to the total weight of the composition.

## Patentansprüche

1. Verwendung von 2-Ethylhexyl-α-cyano-β,β-diphenylacrylat in oder für die Herstellung von kosmetischen Zusammensetzungen, die p-Methylbenzylidencampher und ein Dibenzoylmethanderivat enthalten, zur Verbesserung der Lichtstabilität des p-Methylbenzylidencamphers in diesen Zusammensetzungen.

2. Verwendung nach Anspruch 1, **dadurch gekennzeichnet, daß** es sich bei dem Dibenzoylmethanderivat um 4-tert-Butyl-4'-methoxydibenzoymethan handelt.

3. Verwendung nach Anspruch 1, **dadurch gekennzeichnet, daß** es sich bei dem Dibenzoylmethanderivat um 4-Isopropyldibenzoylmethan handelt.

4. Verwendung nach einem der Ansprüche 1 bis 3, **dadurch gekennzeichnet, daß** das 2-Ethylhexyl-α-cyano-β,β-diphenylacrylat in der Zusammensetzung in einem Anteil von mindestens 0,5 Gew.-%, bezogen auf das Gesamtgewicht der Zusammensetzung, enthalten ist.

5. Verwendung nach Anspruch 4, **dadurch gekennzeichnet, daß** dieser Anteil 0,5 bis 3 Gew.-%, bezogen auf das Gesamtgewicht der Zusammensetzung, beträgt.
